# EUROPEAN PATENT APPLICATION

(11) **EP 1 488 689 A1**
(43) Date of publication of application: **22.12.2004**
(21) Application number: 03743509.6
(22) Date of filing: 26.02.2003
(51) Int. Cl.: A01H 4/00

(54) **METHOD OF PRODUCING CYCLAMEN ROOT TUBER AND METHOD OF PROLIFERATING CYCLAMEN USING THE SAME**

(30) Priority: 04.03.2002 JP 2002104506
(71) Applicant: Tokyo University Of Agriculture Educational Corporation, Setagaya-ku, Tokyo 156-0054 (JP)
(72) Inventor: FUJIGAKI, J., c/o Tokyo University of Agriculture, Tokyo 156-0054 (JP)
(74) Representative: Cresswell, Thomas Anthony
(86) International application number: PCT/JP2003/002132
(87) International publication number: WO 2003/073838

(57) **Abstract**

Cyclamen is grown under a germ-free condition, the end portions of some or all of its roots are cut off, and the cyclamen is cultured in a suitable medium to grow root tubers (bulbs) at these portions. The root tubers are cut off and cultured to be germinated and rooted in order to produce the genetically same individuals (clonal plants). Thereby, cyclamen root tubers are produced stably and efficiently, thereby providing clonal plants.

## Description

### Field of the Invention

The present invention relates to a method of producing cyclamen root tubers by forming root tubers(bulb) in roots of cyclamen by a special technique and to a method of propagating clonal plants of cyclamen which are genetically stable, by using the tubers.

### Description of the Prior Art

Although cyclamen is a plant which is cross-pollinated by an insect, its self-pollination is possible artificially. However, as the inbreeding depression appears in cyclamen after repetitions of self-pollination, seeds are generally produced by mating between different plants in the cultivars. Therefore, a so-called pure line which is genetically fixed cannot be obtained. Moreover, when some cultivars of cyclamen are tetraploid, it is extremely difficult to fix the cyclamen genetically.

To solve this problem, vegetative propagation methods have been investigated to obtain individuals showing genetically the same character by culturing a plant tissue of cyclamen. Most of them are propagation methods in which a tissue is cultured to induce a callus and then to regenerate adventitious shoots and adventitious roots from the callus to form a seedling.

As one of the methods, Ando and Murasaki have proposed a method of propagating cyclamen using an etiolated petiole obtained by the spindly growth of a petiole portions in the dark condition(refer to unpatented document 1).

Ohashi et al have reported a method of propagating cyclamen from a sterile seedling obtained by sterilizing a seed (refer to unpatented document 2).

Ichihashi et al have studied a method of inducing a tuber like body by the liquid culture of an adventitious shoots derived on a solid medium as an explant and proposed a method of propagating cyclamen more efficiently than a method in which only a solid medium is used (refer to unpatented document 3).

Further, it has been proposed to obtain a cyclamen tuber like body having a high germination rate by increasing the content of sugar in a liquid medium in the production of a tuber like body through the liquid culture of a petiole of cyclamen (refer to patented document 1).

However, researches conducted by the inventor of the present invention revealed that these methods above mentioned have problems in reproducibility and stability and that it takes about six months to obtain a seedling, even though it were successful to propagate the explants.

It is an object of the present invention to provide a method capable of producing the root tubers(bulbs) of cyclamen stably and efficiently with extremely high reproducibility as compared with above conventional methods and a method of propagating and growing clonal plants of cyclamen using the tubers.
(unpatented document 1)
   "Report by the Faculty of Horticultural Science of Chiba University" prepared by Ando and Murasaki, vol. 32, pp. 1-5, 1983
(unpatented document 2)
   "Report of Research by the Agricultural Laboratory of Tochigi Prefecture" prepared by Ohashi et al., No. 36, pp.93-108, 1989
(unpatented document 3)
   "Report of Research by the Agricultural Research Center of Gifu Prefecture" prepared by Ichihashi et al., No. 5, pp. 1-23, 1992
(patented document 1)
   JP-A 9-313058 (the term "JP-A" as used herein means an "unexamined published Japanese patent application")

### Summary of the Invention

Cyclamen is a plant which germinates and roots from a corm grown from its hypocotyl. There is unknown a method of propagating a clonal plant of cyclamen by using a cyclamen root tuber (bulb). The inventor of the present invention has conducted studies on a method of propagating a clonal plant of cyclamen efficiently and has succeeded in forming many root tubers in roots of cyclamen by a special technique. It is an object of the present invention to provide a method of producing the root tubers based on this finding. It is another object of the present invention to provide a method of propagating and growing a clonal plant of cyclamen stably and efficiently by germinating and rooting this root tuber.

According to the present invention, the above objects can be attained by a method of producing cyclamen root tubers, comprising cutting off the portions of root or all of the roots of cyclamen and culturing the cyclamen in a medium for forming root tubers to form root tubers (bulbs) at the cut portions of the roots.

That is, the present invention is a method of producing cyclamen root tubers, comprising cutting off the end portions of some or all of the roots of cyclamen and culturing the cyclamen in a medium for forming root tubers to form root tubers (bulbs) at the cut portions of the roots.

According to the present invention, preferably, the end portions of some or all of the roots of cyclamen which is germinated and rooted in a test tube under a germ-free condition are cut off, and the cyclamen is cultured in a solid medium or liquid medium containing a phytohormone to grow root tubers at the cut portions of the roots. As a result, the root tubers for clonal plants of cyclamen of interest can be produced stably. This root tuber is cut off from the parent body and cultured in a new medium or soil to be germinated and rooted, thereby making it possible to obtain a genetically stable clonal plant.

### <cyclamen>

Not only cultivars but also wild species may be used as the donor parent of cyclamen which can be used in the method of the present invention. The cultivars of cyclamen include Victoria, Barberg, Pure White, Pastel and Mini Cyclamen. The present invention is not limited to these cultivars.

As the cyclamen may be used sterile seedlings obtained by sowing the sterilized seeds on a Murashige & Skoog medium (may be abbreviated as "MS medium" hereinafter) germinating and rooting them in the medium. Plants which have passed many generations in a similar medium may also be used. Root tubers formed by the method of the present invention may also be used as the next material after they germinates and roots. The materials are not limited to seedlings germinated and rooted, and may be grown plants having many flowers, leaves and stems.

### <formation of root tuber>

In the method of the present invention, the end portions of some or all of the roots of cyclamen are cut off, and the cyclamen is cultured in a suitable solid or liquid medium in that state to grow root tubers at the cut portions.

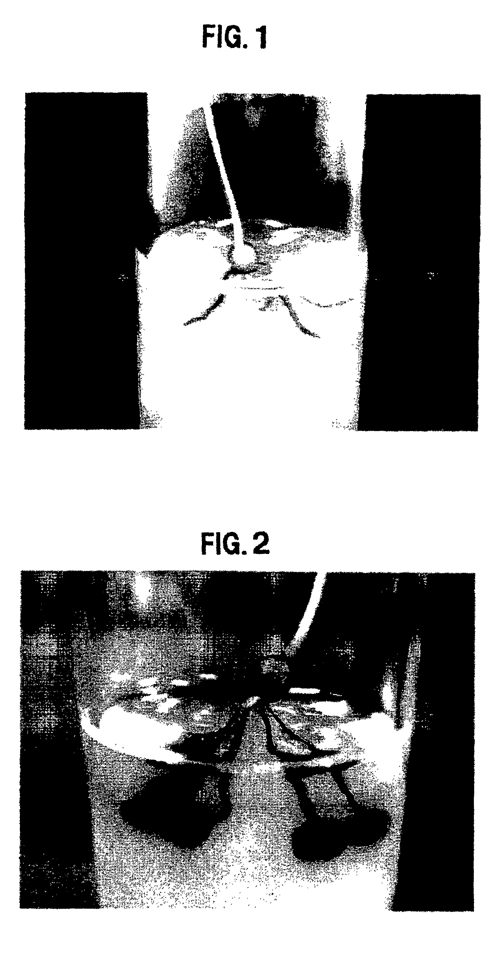

A suitable medium is preferably used to culture the cyclamen. As the medium for forming root tubers may be used a solid medium or liquid medium prepared by adding at least one phytohormone to the above MS medium or a modified MS medium obtained by changing the amount of the major element contained in the MS medium. In the method of the present invention, as shown in Fig. 1, a sterile plant of cyclamen is placed in this medium after the end portions of some or all of its roots are cut off. In general, after almost 30 days of culture, small visible root tubers as shown in Fig. 2 grow at the cut end portions of the roots.

Stationary culture is preferably carried out in the air at 15 to 20°C in the dark. It may also be carried out under weak light or while a culture solution circulates.

The medium for forming root tubers is, for example, a medium prepared by adding at least one phytohormone to a basal culture medium such as Murashige & Skoog medium (abbreviated as MS medium), White medium, Lismaier & Skoog medium or Gamborg B5 medium. Out of these, a medium prepared by adding a specific phytohormone to the MS medium or a modified MS medium obtained by reducing the amount of the major elements out of inorganic salt components including the major element and the minor element contained in the MS medium to 1/3 to 1/2 those of the MS medium is preferred.

The MS medium is a culture medium developed by Toshio Murashige and Folke Skoog for the culture of tobacco in 1962, contains a specific inorganic salt such as NH₄NO₃, KNO₃, CaCl₂, MgSO₄ or KH₂PO₄, and a specific organic substance such as sucrose or myoinositol and is prepared by adjusting its pH to 5.7 to 5.8 and optionally solidfied it with agar. As for its specific constitution and characteristic properties, please refer to "Physiologia Plantarum" vol. 13, pp. 473-497, 1962. The MS medium is widely used directly or after the amount of an inorganic salt as the major element contained in the MS medium is changed, and the latter medium is generally called "modified MS medium".

Examples of the phytohormone to be added to the medium in the method of the present invention include auxins such as α-naphthaleneacetic acid (NAA), 2,4-dichlorophenoxyacetic acid and indoleacetic acid, and cytokinins such as benzylaminopurine (BA), kinetine and zeatin.

Preferred examples of the medium for forming a root tuber are given below.
(i) A modified MS solid medium prepared by changing the amount of the major elements contained in the MS medium to 1/3 to 1/2 those of the MS medium and containing 0.01 to 10 mg/l, preferably 0.1 to 10 mg/l of BA as a phytohormone, 10 to 100 g/l, preferably 30 to 90 g/l of sucrose and 6 to 8 g/l of agar. The medium may further contain 0.01 to 1 mg/l, preferably 0.01 to 0.5 mg/l, more preferably 0.01 to 0.05 mg/l of α-naphthaleneacetic acid (NAA).
(ii) A modified MS liquid medium prepared by changing the amount of the major elements contained in the MS medium to 1/3 to 1/2 those of the MS medium and containing 0.01 to 10 mg/l, preferably 0.1 to 10 mg/l of benzylaminopurine (BA) and 10 to 100 g/l, preferably 30 to 90 g/l of sucrose. The medium may further contain 0.01 to 1 mg/l, preferably 0.01 to 0.5 mg/l, more preferably 0.01 to 0.05 mg/l of α-naphthaleneacetic acid (NAA).

When the above liquid medium is used, it is preferred that the content and temperature of each component in the culture solution should be monitored at all times or at predetermined time intervals and maintained at almost constant values during the culture term by circulating the culture solution. The temperature of the culture solution is preferably adjusted to 15 to 20°C.

It is not preferred that microbes grow in both the solid medium and the liquid medium. Therefore, an antibiotic such as chloramphenicol may be added to this medium.

A root tuber formed by culture using this medium may be germinated and rooted immediately after it is cut off from the parent body. To propagate and grow the root tuber stably, it is preferred that the root tuber should be transferred to a new medium without being cut off and cultured for many generations to be enlarged before it is cut off. The medium used for culture from generation to generation preferably has the same composition as the medium used for germination and rooting which has already been described.

### <Germination and rooting>

The cyclamen root tuber obtained by the above method of the present invention is cut off from the original root of cyclamen and transferred to a suitable medium or soil. After it is cultured for about 30 days to be germinated and rooted, the clonal seedling of cyclamen can be obtained. By growing this, flowers having the same shape and color as the original cyclamen can be reproduced.

Examples of the medium for germination and rooting suitable for use in the culture of this stage are given below.
(i) A modified MS solid medium prepared by changing the amount of the major elements contained in the MS medium to 1/3 to 1/2 those of the MS medium and containing 0.01 to 10 mg/l, preferably 0.01 to 1 mg/l of benzylaminopurine (BA) as a phytohormone, 10 to 100 g/l, preferably 30 to 90 g/l of sucrose and 6 to 8 g/l of agar. The medium may further contain 0.01 to 1 mg/l, preferably 0.01 to 0.5 mg/l, more preferably 0.01 to 0.05 mg/l of α-naphthaleneacetic acid (NAA) as a phytohormone.
(ii) A modified MS liquid medium prepared by changing the amount of the major elements contained in the MS medium to 1/3 to 1/2 those of the MS medium and containing 0.01 to 10 mg/l, preferably 0.01 to 1 mg/l of benzylaminopurine (BA) and 10 to 100 g/l, preferably 30 to 90 g/l of sucrose. The medium may further contain 0.01 to 1 mg/l, preferably 0.01 to 0.5 mg/l, more preferably 0.01 to 0.05 mg/l of α-naphthaleneacetic acid (NAA).

When the above liquid medium is used, the content and temperature of each component in the culture solution can be maintained at almost constant values by circulating the culture solution. Culture in this stage is generally carried out at a temperature of 15 to 20°C under weak light or in the dark (preferably in the dark). It was confirmed that when the liquid medium (ii) is used, the root end portion grows into a root tuber. Further, it was also found that this root tuber can be germinated and rooted by cutting it off from the root and culturing it. This means that a large number of clonal plants of cyclamen can be produced from the root tubers.

### Brief Description of the Drawings

Fig. 1 is a photo of the roots of cyclamen in the stage where end portions of roots of a sterile seedling of cyclamen is planted in a solid medium to be cultured after the end portions of the roots are cut off according to the method of the present invention. More specifically, this is a photo of the roots of cyclamen in the stage where culture is started in a medium containing 50 g/l of sucrose, 1.0 mg/l of BA and 0.05 mg/l of NAA shown in Table 2.
Fig. 2 is a photo of root tubers grown at the end portions of the roots 30 days after the start of the culture.

### Detailed Description of the Preferred Embodiment

A preferred embodiment of the present invention will be described hereinbelow.

In the method of the present invention, after the surface of a cyclamen seed is sterilized with a 1 wt% aqueous solution of sodium hypochlorite, the seed is sown in a culture test tube under a germ-free condition for germination and rooting. When an endogenous germ is existent in the plant, the reproducibility of the present invention degrades. Therefore, it is preferably cultured under a germ-free condition.

### <growth of root tuber>

The end portions of some or all of the roots of the germinated and rooted plant are then cut off, and the plant is placed in a medium in such a manner that at least the cut portions are brought into contact with the medium to be cultured as shown in Fig. 1. After culture is continued for a suitable period of time, as shown in Fig. 2, small root tubers grow at the cut end portions of the roots. The number of roots to be cut and the cutting sites are not particularly limited but the end portions of a plurality of roots are preferably cut off from the viewpoints of productivity and work efficiency. It has been utterly unknown that when the roots are cultured in a suitable medium after the end portions of some or all of them are cut off, root tubers grow at the end portions of the roots, and this was first found by the inventor of the present invention.

The medium for forming root tubers which is suitable for the culture of cyclamen after the end portions of some or all of its roots are cut off is preferably prepared by adding at least one phytohormone to the MS medium or a modified MS medium obtained by reducing the amount of the major elements contained in the MS medium to 1/3 to 1/2 those of the MS medium.

Examples of the phytohormone to be added to the medium include auxins such as NAA(α-naphthaleneacetic acid), 2,4-dichlorophenoxyacetic acid and indoleacetic acid, and cytokinins such as BA(benzylaminopurine), kinetine and zeatin. Out of these, BA is recommended.

A medium containing 0.01 to 10 mg/l, specifically 0.1 to 10 mg/l of BA as a phytohormone is particularly preferred.

The medium may further contain 0.01 to 1 mg/l, preferably 0.01 to 0.5 mg/l, more preferably 0.01 to 0.05 mg/l of α-naphthaleneacetic acid (NAA) as required.

Examples of the carbon source of the medium used for the above culture include sugars such as sucrose and glucose, out of which sucrose is preferred. The content of sucrose is suitably 10 to 100 g/l, preferably 30 to 90 g/l, particularly preferably 50 to 70 g/l. Particularly preferably, this medium contains 6 to 8 g/l of agar to be solidified.

In the method of the present invention, a liquid medium may be used. The liquid medium is preferably a modified MS medium (1/3 MS medium) prepared by reducing the amount of the major elements contained in the MS medium to 1/3 those of the MS medium and containing 10 to 100 g/l, preferably 30 to 90 g/l of sucrose and 0.01 to 10 mg/l, particularly 0.1 to 10 mg/l of BA.

The medium may further contain 0.01 to 1 mg/l, preferably 0.01 to 0.5 mg/l, more preferably 0.01 to 0.05 mg/l of α-naphthaleneacetic acid (NAA) as required.

When cyclamen is cultured in the above medium after the end portions of its roots are cut off, root tubers can be derived.

The pH of the above medium is 5.0 to 8.0, preferably around 7.0. Culture is carried out at 15 to 25° C, preferably 15 to 20°C. Culture may be carried out under illumination (500 to 5,000 lux, preferably 1,000 to 2,000 lux) or in the dark, preferably in the dark.

When cyclamen is cultured for about 30 days under the above conditions, small visible root tubers grow at the cut end portions of the roots as shown in Fig. 2.

### <Germination and rooting>

When the obtained cyclamen root tubers are cut off from the parent body, planted in a new medium (for germination and rooting) and cultured for about 30 days to be germinated and rooted, clonal plants are obtained. Therefore, according to the method of the present invention, clonal plants can be efficiently obtained from the cyclamen seed in about three months.

The medium for germination and rooting used in the culture of this stage may be either a solid medium or a liquid medium.

In the case of a solid medium, for example, it may be the above-described 1/3 MS medium containing 0.01 to 10 mg/l, preferably 0.01 to 1 mg/l of BA, 10 to 100 g/l, preferably 30 to 90 g/l of sucrose and 6 to 8 g/l of agar. The medium may further contain 0.01 to 1 mg/l, preferably 0.01 to 0.5 mg/l, more preferably 0.01 to 0.05 mg/l of NAA.

In the case of a liquid medium, it may be the above-described 1/3 MS medium containing 0.01 to 10 mg/l, preferably 0.01 to 1 mg/l of BA and 10 to 100 g/l, preferably 30 to 90 g/l of sucrose. The medium may further contain 0.01 to 1.0 mg/l, preferably 0.01 to 0.5 mg/l, more preferably 0.01 to 0.05 mg/l of NAA.

When cyclamen is cultured in the above liquid medium after the end portions of its roots are cut off, root tubers can be induced advantageously.

Culture for germination and rooting is generally carried out at room temperature under weak light or in the dark (preferably in the dark).

Then, the thus obtained germinated and rooted root tubers are washed in tap water and gradually acclimated to a natural condition to stand drying as a root tubers plant and may be supplied to cultivators as the root tubers for the clonal plant of cyclamen.

A clonal cyclamen plant can be obtained by planting this root tuber in a field or pot and watering it like the bulb of other plant.

### Examples

The following examples are provided for the purpose of further illustrating the present invention but are in no way to be taken as limiting. The term "1/3 MS medium" means a modified MS medium prepared by reducing the amount of the major elements contained in the MS medium to 1/3 those of the MS medium.

### Example 1 (solid medium)

### <Growth of sterile seedling>

A cyclamen (cultivar: Dressy Scarlet) seed was immersed in an ethyl alcohol aqueous solution having an ethyl alcohol concentration of 70 wt% for 10 seconds and in a sodium hypochlorite solution having an effective chlorine concentration of 1 wt% for 10 minutes to sterilize the surface of the seed. Thereafter, this seed was washed in sterilized water 3 times to remove the sodium hypochlorite from the surface of the seed. This was sown in an MS solid medium containing 30 g/l of sucrose and 6 g/l of agar under a germ-free condition and cultured at a temperature of 20°C under weak light for 30 days to be germinated and rooted.

### <growth of root tuber>

The end portions of the roots of the above germinated and rooted cyclamen seedling were cut off with a scalpel, and the seedling was planted in the following solid media. As the media, a large number of experimental plots were prepared by adding NAA and BA as phytohormones to the 1/3 MS medium in concentrations shown in Tables 1 to 3 and further adding 30 to 90 g/l of sucrose. 6 g/l of agar was added to all of the media which were then sterilized in an autoclave to prepare media having a pH of 7.0. 10 ml of each of the solid media was placed in a 50 ml culture test tube. The stationary culture of the seedling planted in each of the media was carried out at 20° C under weak light for a certain number of days to grow root tubers at the end portions of its roots. The results are shown in Tables 1, 2 and 3.

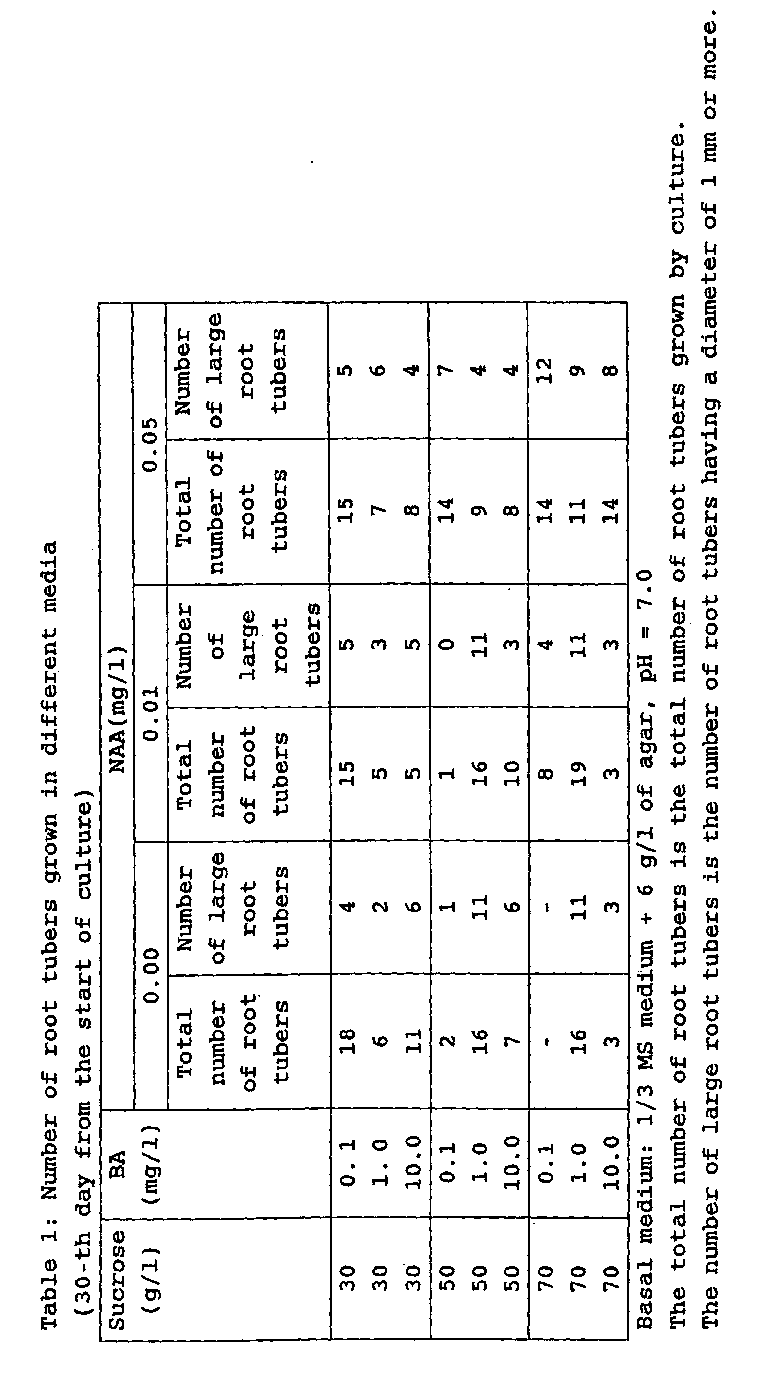

**Table 2:**

| Number of root tubers grown in different media | | | | | |
|---|---|---|---|---|---|
| Sucrose (g/l) | BA (mg/l) | NAA (mg/l) | Number of seedlings planted | Average number of root tubers | |
| | | | | 30-th day | 50-th day |
| 50 | 0.1 | 0.05 | 5 | 2.8 | 2.8 |
| 50 | 1. 0 | 0.05 | 5 | 4.8 | 4.8 |
| 50 | 2. 0 | 0.05 | 5 | 0.6 | 0.6 |
| 50 | 5.0 | 0.05 | 5 | 1.8 | 1.8 |
| 70 | 0.1 | 0.05 | 5 | 4.4 | 4.4 |
| 70 | 1. 0 | 0.05 | 5 | 0.8 | 0.8 |
| 70 | 2.0 | 0.05 | 5 | 3.2 | 3.2 |
| 70 | 5.0 | 0.05 | 5 | 1.0 | 1.0 |
| 90 | 0.1 | 0.05 | 5 | 2.4 | 2.4 |
| 90 | 1. 0 | 0.05 | 5 | 1.6 | 1.6 |
| 90 | 2.0 | 0.05 | 5 | 1.0 | 1.0 |
| 90 | 5.0 | 0.05 | 5 | 0.8 | 0.8 |
| basic medium: 1/3 MS medium + 6 g/l of agar, pH = 7.0 | | | | | |
| The average number of root tubers is the number of root tubers per a seedling. | | | | | |

**Table 3:**

| Influence of medium upon the growth of root tubers (30-th day) | | | | | |
|---|---|---|---|---|---|
| BA (mg/l) | Sucrose (g/l) | Number of seedling planted | Total number of roots cut off | Number of grown root tubers | Enlarging ratio |
| 0.1 | 30 | 5 | 17 | 17 | 2.3 |
| 0.1 | 50 | 5 | 17 | 16 | 3.2 |
| 0.1 | 70 | 5 | 21 | 21 | 3.8 |
| 0.1 | 90 | 5 | 18 | 18 | 5.0 |
| 0.5 | 30 | 5 | 20 | 20 | 6.6 |
| 0.5 | 50 | 5 | 21 | 20 | 9.5 |
| 0.5 | 70 | 5 | 25 | 25 | 7.2 |
| 0.5 | 90 | 5 | 16 | 16 | 6.0 |
| 0. 0 | 30 | 5 | 23 | 23 | 8.4 |
| 1.0 | 50 | 5 | 24 | 23 | 6.3 |
| 1.0 | 70 | 5 | 17 | 17 | 5.4 |
| 1.0 | 90 | 5 | 4 | 3 | 3.8 |
| Basal medium: 1/3 MS + 0.05 mg/l of NAA + 6 g/l of agar, pH = 7.0 | | | | | |
| Total number of roots cut off = total number of roots whose end portions are cut off | | | | | |
| Enlarging ratio = ratio (average value) of the diameter of root tuber when the diameter of the root at the start of culture is 1 | | | | | |

### <germination of root tuber = propagation of clonal plant>

The cyclamen root tuber obtained was cut off from the parent body and planted in an experimental plot prepared by adding 30 to 90 g/l of sucrose, NAA and BA to the 1/3 MS medium as shown in Table 4. 6 g/l of agar was added to the medium which was then sterilized in an autoclave to prepare a solid medium having a pH of 7.0. The culture test tube was left under illumination for 12 hours a day and in the dark for 12 hours. As a result, the root tubers germinated and rooted while enlarging. The experimental results are shown in Table 4.

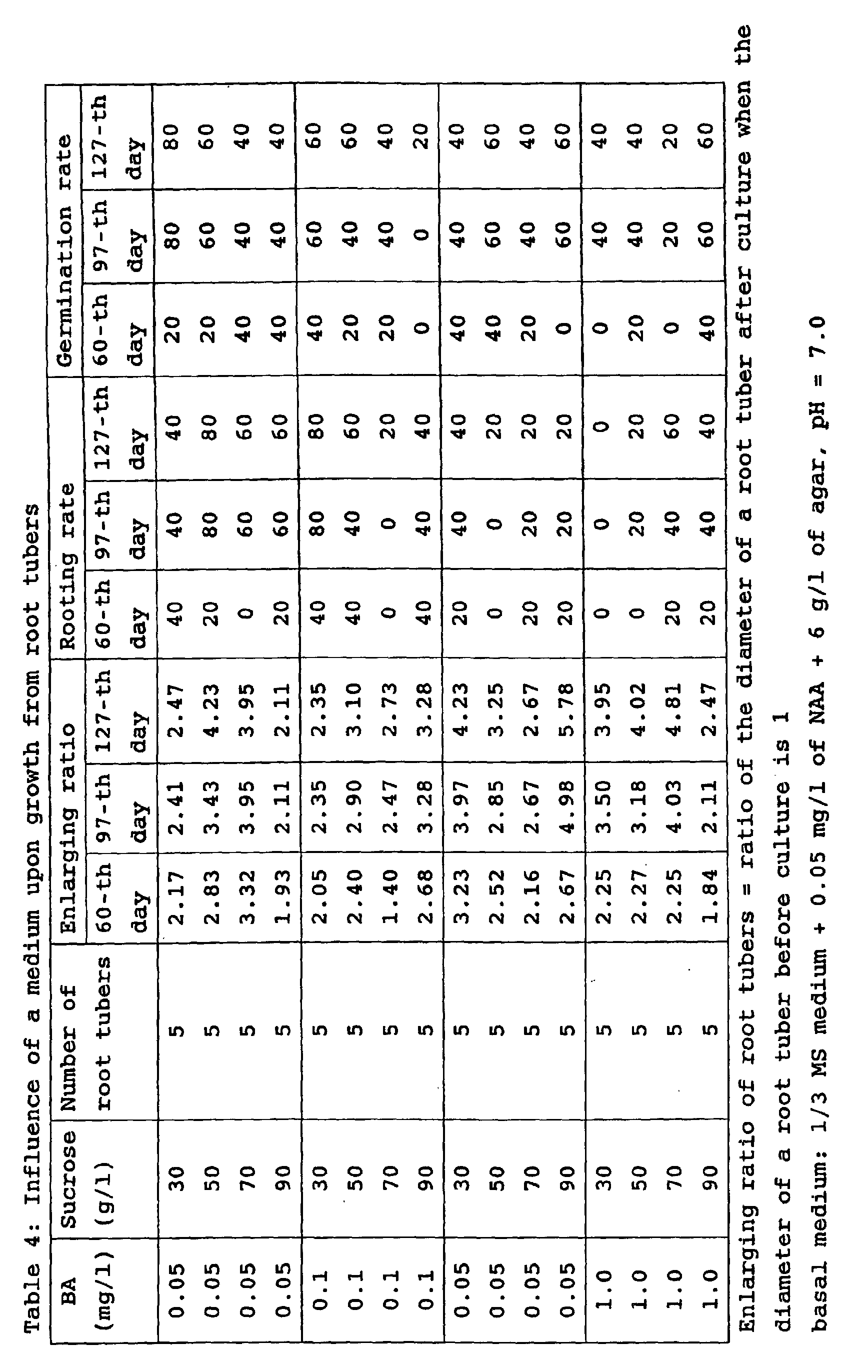

The influence of illumination during culture for germination and rooting was further investigated. That is, 10 root tubers were planted in each of the following four different media A, B, C and D prepared by adding different amounts of BA and sucrose to the following basal medium to be investigated.
Basal medium: 1/3 MS medium + 0.05 mg/l of NAA + 6 g/l of agar, pH = 7.0
Medium A: 0 mg/l of BA and 90 g/l of sucrose
Medium B: 0.05 mg/l of BA and 30 g/l of sucrose
Medium C: 0.5 mg/l of BA and 90 g/l of sucrose
Medium D: 0.5 mg/l of BA and 50 g/l of sucrose

Germination and rooting were observed by using the above media and illuminating right above them at 1,500 lux for a certain time each day. The illumination time "0" in Table 5 means that the root tuber was cultured in the dark. As a result, culture in the dark is the most effective as obvious from Table 5.

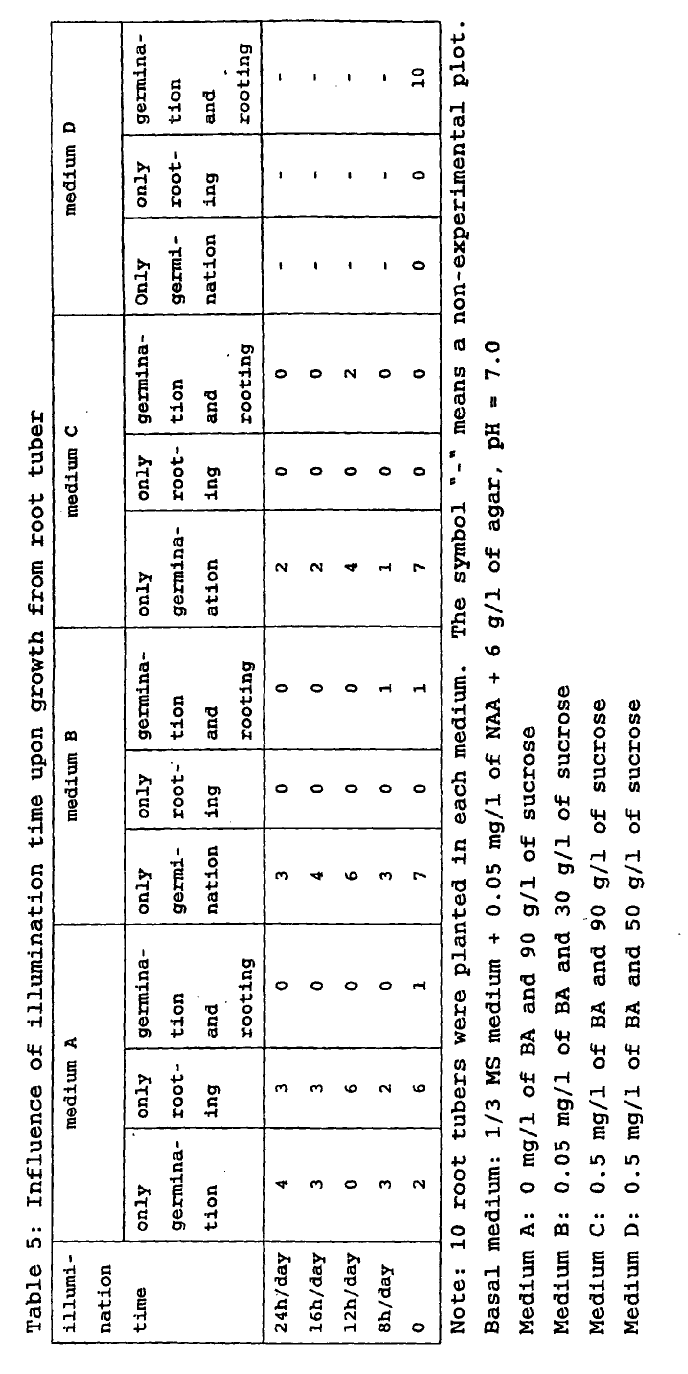

### Comparative Example 1 (culture of explants from leaves and petiole)

For comparison, the explants from leaves and petioles of cyclamen (species: Flamenco) were cultured for 30 days by changing the contents of BA and sucrose in a basal medium prepared by adding 0.05 mg/l of NAA and 6 g/l of agar to the 1/3 MS medium and having a pH of 7.0. The results are shown in Table 6. In all the cases, the root tubers were hardly enlarged by culture and rarely regenerated and accordingly, a clonal plant could not be obtained with high reproducibility.

**Table 6:**

| Enlargement and a number of regeneration of the explants from leaves and petioles of Cyclamen plants on different media | | | | | | | |
|---|---|---|---|---|---|---|---|
| BA (mg/l) | Sucrose (g/m) | Explants from leaves | | | Explants from petioles | | |
| | | Number of explants planted | Number of enlarged explants | Number of regenerate explants | Number of explants planted | Number of enlarged explants | Number of regenerate explants |
| 0.05 | 30 | 20 | 1 | 0 | 20 | 2 | 0 |
| 0.05 | 50 | 20 | 0 | 0 | 20 | 1 | 0 |
| 0.05 | 70 | 20 | 0 | 0 | 20 | 1 | 0 |
| 0.05 | 90 | 20 | 0 | 0 | 20 | 0 | 0 |
| 0.1 | 30 | 20 | 2 | 1 | 20 | 4 | 4 |
| 0.1 | 50 | 20 | 1 | 0 | 20 | 5 | 1 |
| 0.1 | 70 | 20 | 0 | 0 | 20 | 2 | 0 |
| 0.1 | 90 | 20 | 0 | 0 | 20 | 1 | 0 |
| 0.5 | 30 | 20 | 2 | 2 | 20 | 3 | 1 |
| 0.5 | 50 | 20 | 4 | 1 | 20 | 4 | 1 |
| 0.5 | 70 | 20 | 1 | 0 | 20 | 1 | 0 |
| 0.5 | 90 | 20 | 2 | 0 | 20 | 0 | 0 |
| 1 | 30 | 20 | 6 | 3 | 20 | 1 | 0 |
| 1 | 50 | 20 | 4 | 2 | 20 | 1 | 10 |
| 1 | 70 | 20 | 3 | 1 | 20 | 0 | 0 |
| 1 | 90 | 20 | 5 | 1 | 20 | 0 | 0 |
| Basal medium: 1/3 MS medium + 0.05 mg/l of NAA + 6 g/l of agar, pH = 7.0 | | | | | | | |

### Example 2 (liquid medium)

Cyclamen seeds were sown in a phytohormone-free 1/3 MS medium under a germ-free condition, the end portions of the roots of the germinated 40-day seedlings were cut off with a knife, and the seedlings were cultured in four different liquid media shown in Table 7 which differed from one another in the contents of an inorganic salt, sucrose and NAA and BA as phytohormones for 30 days. A float was wound round the corm and rotational culture was carried out while the corm floated in the liquid medium.

As a result, as shown in Table 7, the root tuber formation rate was 100 % in the 1/3 MS medium containing 50 g/l of sucrose and 0.5 mg/l of BA. When these two media were compared with each other, it was found that the root tubers were enlarged more in an NAA-free medium than in an NAA-added medium.

Further, it was found that the root tubers can be induced even in media containing no inorganic salt in 30 days of a test period if sucrose and BA are added to the medium. However, the root tubers are not enlarged so much under these conditions.

As a result, it was confirmed that the root tubers can be induced at the cut ends of cyclamen roots in liquid media like media which are solidified with agar.

**Table 7:**

| Effects of medium components upon growth of root tuber in liquid medium (30-th day of culture) | | | | | | |
|---|---|---|---|---|---|---|
| Name of plot | Inorganic salt | Sucrose (g/l) | NAA (mg/l) | BA (mg/l) | Root tuber production rate (%) | Productive rate of large root tube (≧3mm) |
| a | 0 | 50 | 0 | 0.5 | 32 | 0 |
| b | 0 | 50 | 0.05 | 0.5 | 75 | 0 |
| c | 1/3MS | 50 | 0 | 0.5 | 100 | 30 |
| d | 1/3MS | 50 | 0.05 | 0.5 | 100 | 16 |
| Note: "0" in the column of "inorganic salt" in Table 7 means a medium containing no inorganic salt, and "1/3MS" means a modified MS medium prepared by reducing the amount of the major elements contained in MS medium to 1/3 that of the MS medium. | | | | | | |

As obvious from Examples 1 and 2, it was confirmed that when the end portions of the roots of cyclamen are cut off and the cyclamen is cultured in the 1/3 MS solid medium containing sucrose and BA or a modified MS liquid medium prepared by reducing the amount of the major elements to 1/3 to 1/2 those of the MS medium and containing 0.01 to 10 mg/l of BA and 10 to 100 g/l of sucrose, the end portions of the roots grow into root tubers. It was also found that when each of the root tubers is cut off from the root and cultured, it can be germinated and rooted. This means that a large number of clonal individuals of cyclamen can be produced from the root tubers.

### Effect of the Invention

According to the present invention, there is provided a method of producing cyclamen which is a plant requiring much time and labor stably and efficiently with high reproducibility. That is, according to the method of producing the root tubers of cyclamen of the present invention, the root tubers of cyclamen which are genetically stable can be produced with high reproducibility. This method can be applied to any cyclamen species (including cultivars and wild species).

According to the method of propagating cyclamen of the present invention, clonal plants can be obtained in about 3 months at the shortest and the cyclamen clonal seedlings which are genetically stable can be obtained more efficiently than the method of the prior art.

### Industrial Feasibility

Since clonal seedlings of cyclamen obtained by the present invention are uniform and the root tubers are of great product value at the time of shipping, the method of the present invention is suitable for the commercial-scale production of cyclamen.

## Claims

1. A method of producing cyclamen root tubers, comprising cutting off the portions of roots or all of roots of cyclamen and culturing the cyclamen in a medium for forming root tubers and forming root tubers (bulbs) at the cut portions.

2. The method of producing cyclamen root tubers according to claim 1, wherein a plant obtained by sowing a cyclamen seed under a germ-free condition and germinating and rooting it is used as the cyclamen.

3. The method of producing cyclamen root tubers according to claim 1, wherein a Murashige & Skoog medium (MS medium) or a medium prepared by adding a phytohormone to a modified MS medium is used as the medium for forming root tubers.

4. The method of producing cyclamen root tubers according to claim 3, wherein a modified MS solid medium prepared by reducing the amount of the major elements contained in the MS medium to 1/3 to 1/2 those of the MS medium and containing 0.01 to 10 mg/l of benzylaminopurine (BA), 10 to 100 g/l of sucrose and 6 to 8 g/l of agar is used as the medium for forming root tubers.

5. The method of producing cyclamen root tubers according to claim 3, wherein a modified MS liquid medium prepared by reducing the amount of the major elements contained in the MS medium to 1/3 to 1/2 those of the MS medium and containing 0.01 to 10 mg/l of benzylaminopurine (BA) and 10 to 100 g/l of sucrose is used as the medium for forming root tubers.

6. A method of propagating cyclamen, comprising obtaining clonal plants by germinating and rooting the cyclamen root tubers formed by the method of claim 1.

7. The method of propagating cyclamen according to claim 6, wherein the cyclamen root tubers root is germinated and rooted in a medium for germination and rooting.

8. The method of propagating cyclamen according to claim 7, wherein a modified MS liquid medium prepared by reducing the amount of the major elements contained in the MS medium to 1/3 to 1/2 those of the MS medium and containing 0.01 to 10 mg/l of benzylaminopurine (BA) and 10 to 100 g/l of sucrose is used as the medium for germination and rooting.

9. The method of propagating cyclamen according to claim 7, wherein a modified MS solid medium prepared by reducing the amount of the major elements contained in the MS medium to 1/3 to 1/2 those of the MS medium and containing 0.01 to 10 mg/l of benzylaminopurine (BA), 10 to 100 g/l of sucrose and 6 to 8 g/l of agar is used as the medium for germination and rooting.
